# EUROPEAN PATENT APPLICATION

(11) **EP 4 834 920 A1**
(43) Date of publication of application: **04.11.2026**
(21) Application number: 24911525.4
(22) Date of filing: 27.12.2024
(51) Int. Cl.: C07K 14/47, C07K 16/00, C12N 15/00, C12N 5/00

(54) **METHOD FOR CONSTRUCTING CELL WHICH PRODUCES DEFUCOSYLATED PROTEIN AND USE OF CELL**

(30) Priority: 28.12.2023 WO PCT/CN2023/142729
(71) Applicant: Wuxi Biologics Ireland Limited, Dundalk, Co Louth A91 X56F (IE)
(72) Inventor: ZHOU, Yangfang, Shanghai 201208 (CN); LI, Ying, Shanghai 201208 (CN); RAO, Yifan, Shanghai 201208 (CN); GAO, Rong, Shanghai 201208 (CN); ZHU, Xiaobin, Shanghai 201208 (CN); WANG, Lile, Shanghai 201208 (CN); LIAO, Shanhui, Shanghai 201208 (CN); WANG, Shuai, Shanghai 201208 (CN); ZHANG, Yu, Shanghai 201208 (CN); ZHANG, Sheng, Shanghai 201208 (CN)
(74) Representative: Modiano, Gabriella Diana
(86) International application number: PCT/CN2024/143321
(87) International publication number: WO 2025/140609

(57) **Abstract**

Provided is a method for constructing a cell which produces defucosylated protein using a zinc finger nuclease which contains a zinc finger protein capable of binding to the FUT8 gene in a targeted manner and a DNA cleavage domain or cleavage half-domain. Also provided are a polynucleotide which encodes the zinc finger protein and the zinc finger nuclease, and cells containing the polynucleotide and the zinc finger nuclease.

## Description

### TECHNICAL FIELD

The present disclosure relates to the field of molecular biology and biopharmaceuticals; in particular, to a cell for producing a defucosylated protein constructed by gene editing using zinc finger nuclease technology, and the use thereof.

### BACKGROUND ART

In recent years, with the successful preparation and wide application of genetically engineered antibodies, and the significant improvement of antibody production levels, the research and development of antibody drugs has entered a new stage of rapid development. According to literature reports, by December 2019, there were 79 therapeutic antibodies on the market, which were mainly used for tumors, autoimmune diseases, metabolic diseases and infectious diseases (Lu RM et al. (2020) J Biomed Sci. 27(1):1-30). The top ten global best-selling anti-tumor drugs in 2022 included four antibody drugs, whose combined sales reached nearly US$42.492 billion.

Antibodies activate the immune system to destroy and attack cells through two mechanisms: complement-dependent cytotoxicity (CDC) and antibody-dependent cellular cytotoxicity (ADCC). ADCC is an immune response generated mainly by natural killer (NK) cells against antibody-coated targets. In ADCC, NK cells recognize the constant (Fc) region of an antibody mainly via interaction with the FcγRIII receptor on NK cells. Then, NK cells deposit perforin and granzymes on the surface of target cells, inducing cell lysis and apoptosis, respectively.

Most mammalian immunoglobulins are fucosylated, including immunoglobulins produced by Chinese hamster ovary cells (CHO cells). α-1,6-fucosyltransferase (FUT8) catalyzes the transfer of a fucose residue from guanosine diphosphate-fucose (GDP-Fuc) to the terminus of the innermost N-acetylglucosamine (GlcNAc) of the core of an N-glycan to form fucose attached to the core region of the antibody Fc. Studies have shown that, when fucose is removed from the glycan structure at position Asn297 of the CH2 domain of an IgG antibody Fc fragment, the binding between NK cell FcγRIIIa and the IgG antibody Fc fragment can be enhanced, thereby enhancing the ADCC effect of the antibody (Shinkawa T et al. (2003) J Biol Chem. 278(5):3466-73).

At present, there are three common methods for industrially producing defucosylated antibodies: (1) adding a fucosylation inhibitor, such as 2-fluorofucose (2FF), to a culture medium (Rillahan CD et al. (2012) Nat Chem Biol. 8(7):661-8); (2) stably overexpressing a bacterial RMD protein (GDP-6-deoxy-D-lyxo-4-hexulose reductase) in engineered cells (von Horsten HH et al. (2010) Glycobiology. 20(12):1607-18), thereby blocking fucosylation modification of the Fc region by interfering with the formation of the substrate GDP-fucose; and (3) interfering with key genes in the fucose synthetic pathway, such as introducing the expression of β(1,4)-N-acetylglucosaminyltransferase III (GnTIII) (Popp O et al. (2018) MAbs. 10(2):290-303), knocking out the GDP-fucose transporter (GFT) (Omasa T et al. (2008) J Biosci Bioeng. 106(2):168-73), or knocking out the α-1,6-fucosyltransferase (FUT8) (Yamane-Ohnuki N et al. (2004) Biotechnol Bioeng. 87(5):614-22).

There remains a need in the art for a new method for constructing cells for producing defucosylated proteins.

### SUMMARY OF THE INVENTION

To meet the above need, after long-term and in-depth exploration and research, the inventors have developed a new method for preparing cells for producing defucosylated proteins. This method uses a zinc finger nuclease to inactivate the FUT8 gene in a cell, and the resulting cell produces a defucosylated protein (e.g., an antibody) having strong effector function, which has broad industrial application prospects.

In one aspect, provided herein is a zinc finger protein capable of targeting and binding to the FUT8 gene, which comprises, sequentially from the N-terminus to the C-terminus, amino acid sequences of zinc finger recognition regions of zinc fingers F1 to F5 or F1 to F6 shown in the same row of the following table:

| F1 | F2 | F3 | F4 | F5 | |
|---|---|---|---|---|---|
| SKWNLRS (SEQ ID NO: 24) | AQSNLLS (SEQ ID NO: 25) | LRHQLRR (SEQ ID NO: 26) | RSDYLTN (SEQ ID NO: 17) | RSDYLTN (SEQ ID NO: 17) | |

| F1 | F2 | F3 | F4 | F5 | |
|---|---|---|---|---|---|
| RADNLTE (SEQ ID NO: 30) | TSGNLTE (SEQ ID NO: 31) | TSGHLVR (SEQ ID NO: 32) | RKDNLKN (SEQ ID NO: 33) | RKDNLKN (SEQ ID NO: 33) | |

| F1 | F2 | F3 | F4 | F5 | |
|---|---|---|---|---|---|
| DRSNLLS (SEQ ID NO: 39) | NQSNLLR (SEQ ID NO: 40) | FHSNLLA (SEQ ID NO: 18) | QLSTLNY (SEQ ID NO: 41) | QSGNLSR (SEQ ID NO: 42) | |

| F1 | F2 | F3 | F4 | F5 | F6 |
|---|---|---|---|---|---|
| QLATLNR (SEQ ID NO: 13) | TRWNLRA (SEQ ID NO: 14) | SRRDLRR (SEQ ID NO: 15) | WRRRLLS (SEQ ID NO: 16) | RSDYLTN (SEQ ID NO: 17) | FHSNLLA (SEQ ID NO: 18) |

| F1 | F2 | F3 | F4 | F5 | F6 |
|---|---|---|---|---|---|
| RKSHLTM (SEQ ID NO: 19) | FHSGLLA (SEQ ID NO: 20) | WRRRLLS (SEQ ID NO: 16) | RKYVLLR (SEQ ID NO: 21) | RKDYLVL (SEQ ID NO: 22) | QQAGLIN (SEQ ID NO: 23) |

| F1 | F2 | F3 | F4 | F5 | F6 |
|---|---|---|---|---|---|
| RSDYLTN | QKITLVR | RSDYLTN | QLATLNR | SRFNLTR | TKYILTN |
| (SEQ ID NO: 17) | (SEQ ID NO: 27) | (SEQ ID NO: 17) | (SEQ ID NO: 13) | (SEQ ID NO: 28) | (SEQ ID NO: 29) |

| F1 | F2 | F3 | F4 | F5 | F6 |
|---|---|---|---|---|---|
| RSDNLSV (SEQ ID NO: 34) | SPADLTR (SEQ ID NO: 35) | RSDHLSQ (SEQ ID NO: 36) | QSGDLRR (SEQ ID NO: 37) | RSDNLVR (SEQ ID NO: 38) | RKDNLKN (SEQ ID NO: 33) |

| F1 | F2 | F3 | F4 | F5 | F6 |
|---|---|---|---|---|---|
| FHSGLLA (SEQ ID NO: 20) | HPSTLSK (SEQ ID NO: 43) | ARWTLDC (SEQ ID NO: 44) | HPSTLSK (SEQ ID NO: 43) | HPSTLSK (SEQ ID NO: 43) | RKFTLTN (SEQ ID NO: 45) |

In another aspect, provided herein is a zinc finger nuclease (ZFN) targeting the FUT8 gene, which is a fusion protein comprising the ZFP described herein and at least one DNA cleavage domain or cleavage half-domain.

In yet another aspect, provided herein is a polynucleotide encoding the ZFP or ZFN described herein.

In another aspect, provided herein is a vector comprising the polynucleotide as described above.

In another aspect, provided herein is an isolated cell (e.g., a host cell) comprising the ZFP, ZFN, polynucleotide or vector described herein.

In another aspect, provided herein is a cell line in which the FUT8 gene is inactivated or partially inactivated, which comprises the ZFP, ZFN, polynucleotide or vector described herein.

In another aspect, provided herein is a method for producing a FUT8-deficient cell, the method comprising:
(a) introducing into a cell a polynucleotide encoding one or more ZFNs, wherein each of the ZFNs comprises: (i) the ZFP described herein, which is capable of binding to a target site in the FUT8 gene in the cell; and (ii) a DNA cleavage domain or cleavage half-domain;
(b) allowing the ZFN to be expressed in the cell, such that the ZFN binds to the target site and cleaves the FUT8 gene.

In another aspect, provided herein is a method for producing a FUT8-deficient cell line, the method comprising:
(a) inactivating endogenous FUT8 gene in a cell by the method for producing a FUT8-deficient cell as described herein; and
(b) culturing the cell under conditions suitable for producing a FUT8-deficient cell line.

In yet another aspect, provided herein is a method for producing a recombinant protein of interest in a host cell, the method comprising:
(a) providing the FUT8-deficient cell described herein;
(b) introducing into the cell an expression vector comprising a nucleic acid encoding the recombinant protein of interest, thereby producing the recombinant protein of interest.

In another aspect, provided herein is a method for producing a recombinant protein of interest in a host cell, the method comprising:
(a) providing a cell comprising endogenous FUT8 gene;
(b) inactivating the endogenous FUT8 gene in the cell by the method for producing a FUT8-deficient cell as described herein; and
(c) introducing into the cell an expression vector comprising a nucleic acid encoding the recombinant protein of interest, thereby producing the recombinant protein of interest.

In another aspect, provided herein is the use of the ZFP, ZFN, polynucleotide or vector described herein for producing a FUT8-deficient cell.

In another aspect, provided herein is the use of the ZFP, ZFN, polynucleotide, vector, cell or cell line described herein for producing a defucosylated Fc-containing recombinant protein.

In another aspect, provided herein is the use of the ZFP, ZFN, polynucleotide, vector, cell or cell line described herein for producing an antibody (such as a monoclonal antibody) capable of inducing an enhanced ADCC effect.

In another aspect, provided herein is a FUT8-deficient cell line prepared according to the method described herein for producing a FUT8-deficient cell line.

In another aspect, provided herein is a FUT8-deficient cell line, which comprises at the FUT8 locus a nucleotide sequence shown in one or more of SEQ ID NO: 52, SEQ ID NO: 53, SEQ ID NO: 54, SEQ ID NO: 55, SEQ ID NO: 56, SEQ ID NO: 57, SEQ ID NO: 58 and SEQ ID NO: 59.

In another aspect, provided herein is a FUT8-deficient cell line comprising an engineered FUT8 locus, wherein the engineered FUT8 locus can be detected by the following primer pairs:
Primer pair (i):
   Forward primer: AGCCTGAAGTACATAGCCGA (SEQ ID NO: 46); and
   Reverse primer: TGCCACTGCTTCTATATACTGATTC (SEQ ID NO: 47); and/or
Primer pair (ii):
   Forward primer: GACGCACTGACAAAGTGGGA (SEQ ID NO: 48); and
   Reverse primer: GGTCTGTTCCATCCCCAGAATG (SEQ ID NO: 49);
   and/or
Primer pair (iii):
   Forward primer: CTGTTGATTCCAGGTTCCCA (SEQ ID NO: 50); and
   Reverse primer: TGTTACTTAAGCCCCAGGC (SEQ ID NO: 51).

In another aspect, provided herein is a method for culturing a FUT8-deficient cell line, comprising:
(a) providing the FUT8-deficient cell line according to any one of claims 15-17; and
(b) culturing or expanding the cells of the cell line.

### Brief Description of the Drawings

The present invention is further described below with reference to the accompanying drawings, wherein these drawings are provided merely to illustrate embodiments of the present invention, and are not intended to limit the scope of the present invention.

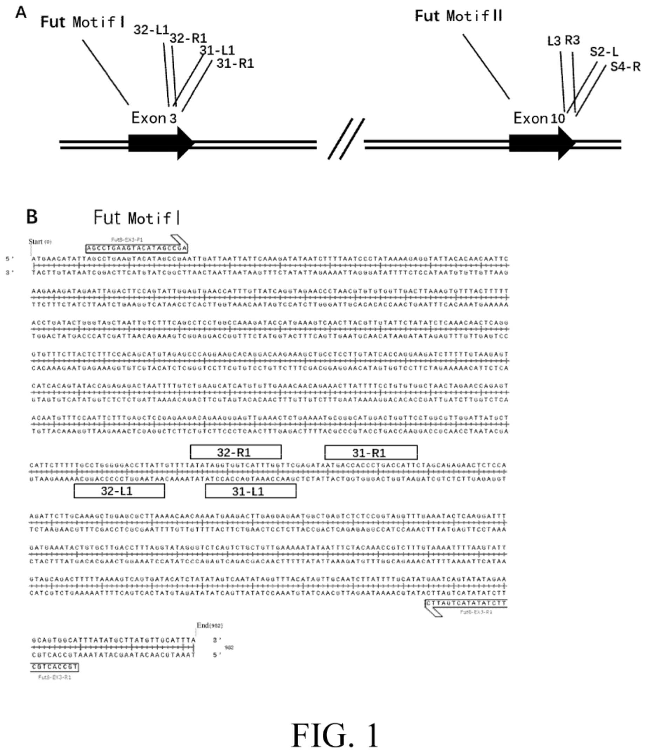

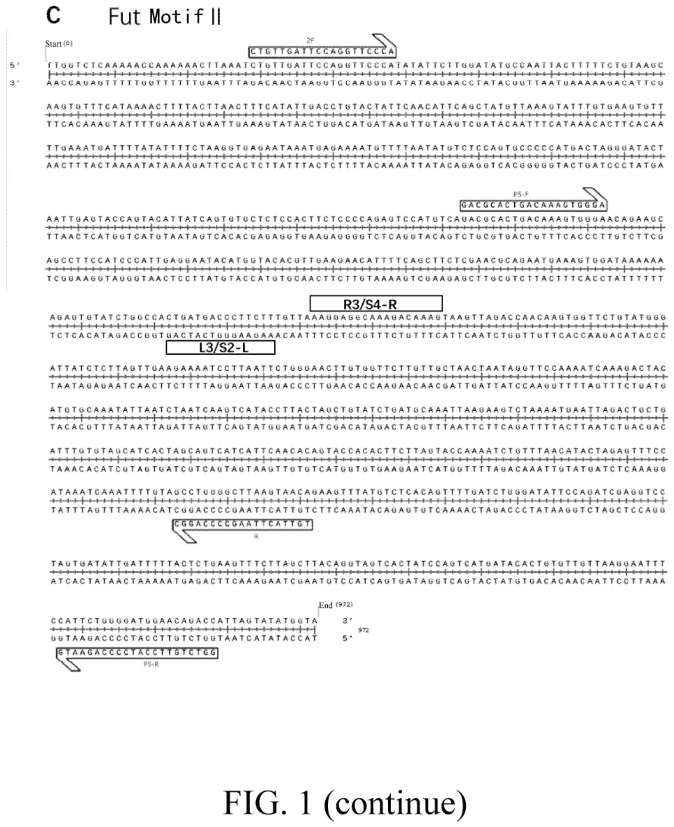

Figure 1 shows the binding sites, cleavage sites, and PCR primer sites of zinc finger nucleases targeting FUT8 exon 3 and exon 10 and regions adjacent thereto according to an exemplary embodiment disclosed herein.

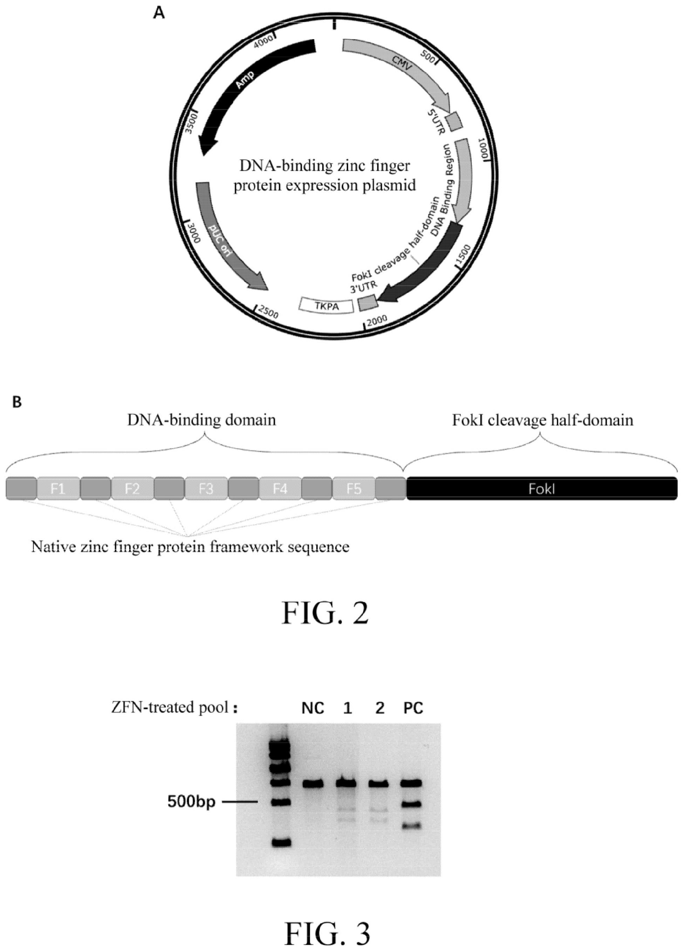

Figure 2A shows a schematic diagram of an expression plasmid map of a zinc finger nuclease according to an exemplary embodiment disclosed herein. The zinc finger protein and a cleavage half-domain of the restriction endonuclease FokI are fused and cloned into a plasmid. The fusion protein is under the control of a CMV promoter. Figure 2B shows a schematic diagram of the specific structure of a zinc finger nuclease according to an exemplary embodiment disclosed herein, wherein the respective zinc finger binding domains are inserted in order between framework sequences of a native zinc finger protein, and are linked at the C-terminus to a FokI cleavage half-domain.
Figure 3 shows that a T7E1 digestion assay verifying whether the ZFN plasmids effectively excise the target gene sequence. Lane 1: S2-L and S4-R transfection, with an editing efficiency of 4.7% calculated based on band intensity measured by ImageJ; Lane 2: L3 and R3 transfection, with an editing efficiency of 6.2%; NC: negative control group, namely a genomic DNA of cells in which the FUT8 gene was not knocked out; PC: positive control group, namely a cell genome in which FUT8 gene has been knocked out at a proportion of 50%.
Figure 4 shows results of flow cytometric analysis of a cell pool stained with green fluorescence-labeled Lens culinaris agglutinin (LCA-FITC). (A) Quantitative analysis of different subpopulations of cells, wherein the circled cell population with lower green fluorescence represents the FUT8^{-/-} cell population, accounting for 27.44% of the cell pool. (B) Peak shifts of different subpopulations of cells.
Figure 5 shows flow cytometry analysis results of clone 154-F8ZFN-09-08 and a negative control after LCA-FITC staining. Solid line: 154-F8ZFN-09-08 cells; dashed line: negative control in which the FUT8 gene was not knocked out.
Figure 6 shows NGS verification results demonstrating that both alleles of clones 154-F8ZFN-09-08 and 154-F8ZFN-10-02 were knocked out.

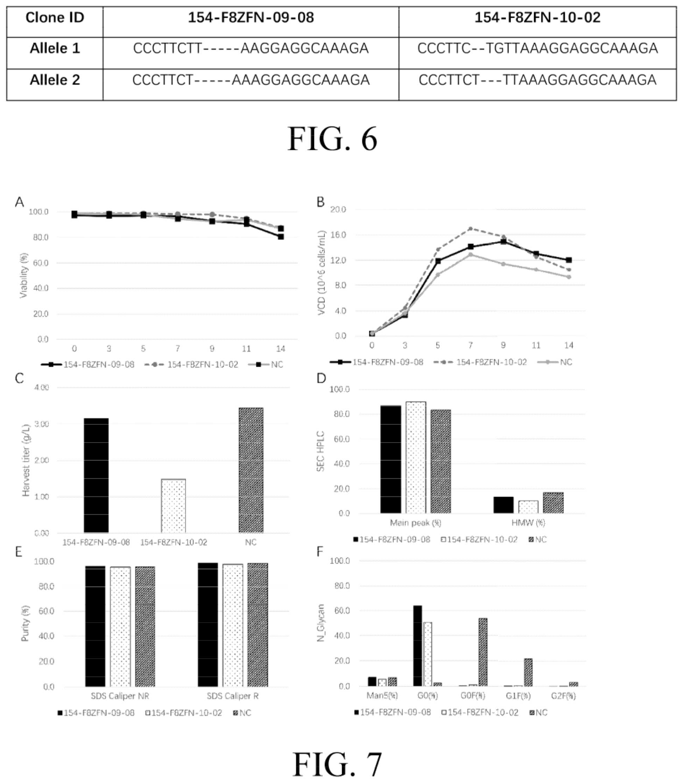

Figure 7 shows the application of FUT8^{-/-} cell lines (i.e., 154-F8ZFN-09-08 and 154-F8ZFN-10-02) in monoclonal antibody expression screening. NC: negative control in which the FUT8 gene was not knocked out. (A) Change in viability during FB culture after recovery of the cell pool; (B) change in viable cell density (VCD) during FB culture of the cell pool; (C) protein expression titer on day 14 during fed-batch culture; (D) size-exclusion chromatography (SEC-HPLC) analysis after one-step ProA purification of the harvested supernatant; (E) SDS Caliper NR and SDS Caliper R analysis of one-step purified samples; (F) glycoform profiles of one-step purified samples.
Figure 8 shows performance evaluation results of a FUT8^{-/-} cell strain (154-F8ZFN-09-08) in antibody production. (A) Cell viability of the 154-F8ZFN-09-08 group during FB culture; (B) change in viable cell density (VCD); (C) protein expression titer; (D) SEC-HPLC and SDS Caliper NR analysis results; (E) glycoform analysis results.
Figure 9 shows the results of the gene editing efficiency assay. Lane 1: 31-L1/31-R1 knockout sample, with an editing efficiency of 9.4%; Lane 2: 32-L1/32-R1 knockout sample, with an editing efficiency of 10.9%; NC: wild-type CHO-K1.
Figure 10 shows SCP monoclonal sorting results. The unstained sample represents CHO K1; Fut8^{-/-} clone C6 represents a monoclone selected by LCA-FITC staining subsequent to single-cell sorting; Fut8 wt represents LCA-FITC staining of CHO K1 cells.
Figure 11 shows SCP monoclonal sorting results. The unstained sample represents CHO K1-N079; Fut8^{-/-} clone F2 represents a monoclone selected after LCA-FITC staining following sorting; Fut8 wt represents LCA-FITC staining of CHO K1 cells.
Figure 12 shows NGS verification results demonstrating that one pair of alleles of clones C6 and F2 were knocked out.
Figure 13 shows that the ADCC effect of adalimumab produced by FUT8-knockout host cells was significantly enhanced compared with a wild-type control.

### Detailed Description of the Embodiments

In the present application, the meanings of scientific and technical terms have the same meanings as commonly understood by one of ordinary skill in the art, unless otherwise specified. In the present application, "a/an" or a combination thereof with various quantifiers includes both singular and plural meanings, unless specifically stated otherwise. In the present application, with respect to the same parameter or variable, when multiple numerical values, numerical ranges, or combinations thereof are provided, this is deemed to specifically disclose these numerical values, the endpoints of the ranges, and numerical ranges formed by any combination thereof. In the present application, any numerical value, whether or not modified by a modifier such as "about", invariably encompasses an approximate range that can be understood by those skilled in the art, for example **±**10%, **±**5%, and the like. As used herein, each reference to an "embodiment" equally applies to and encompasses the methods and systems of the present application. In the present application, one or more technical features in any embodiment may be freely combined with one or more technical features in any one or more other embodiments, and the embodiments obtained thereby likewise fall within the scope of the present disclosure.

In mammalian cells, Fut8 attaches core fucose to oligosaccharides present on the Fc region of an antibody, which is generally considered to be very important for the effector function of antibody-dependent cellular cytotoxicity. Three-dimensional analysis of the human Fut8 structure shows that three α2/α6 fucosyltransferase motifs form the catalytic core of the enzyme. See Ihara et al. (2007) Glycobiology 17:455-66. In this region, point mutations of many individual residues to alanine result in complete inactivation of the enzyme. See Ihara et al. (2007) Glycobiology 17:455-66; Takahashi et al. (2000) Glycobiology 10:503-10. Studies have shown that cells in which the expression of FUT8 is reduced or eliminated (for example, in knockout cell lines or cells treated with siRNA) can produce non-fucosylated antibodies having stronger effector function. See, for example, Kanada et al. (2007) Biotechnol. 130(3):300-310; Kanada et al. (2007) Glycobiology 18:104-118; Mori et al. (2004) Biotechnol. Bioeng. 88:901-908.

Provided herein is a method for constructing a cell for producing a defucosylated protein, wherein the method uses a zinc finger nuclease targeting the FUT8 gene to inactivate the FUT8 gene in the cell. The zinc finger nuclease (ZFN) comprises a zinc finger protein (ZFP) and a nuclease cleavage domain or cleavage half-domain. The inventors have surprisingly found that, by using the zinc finger nucleases specifically designed as described herein, the FUT8 gene in a cell can be knocked out with high efficiency, and the resulting cell produces a defucosylated protein (for example, an antibody) having stronger effector function.

### A. Zinc Finger Protein

In one aspect, provided herein is a zinc finger protein (ZFP) capable of targeting and binding to the FUT8 gene.

As used herein, the term "zinc finger protein" or "ZFP" refers to a protein that binds DNA in a sequence-specific manner through one or more zinc fingers (or zinc finger recognition regions). A zinc finger is a region of amino acid sequence within the protein, the structure of which is stabilized by coordination of zinc ions.

The ZFP described herein is a non-naturally occurring ZFP. The ZFP may have 1, 2, 3, 4, 5, 6, or more zinc fingers, each of which has a zinc finger recognition region capable of binding to a target site in the FUT8 gene. In some embodiments, the ZFP comprises five or six zinc fingers.

As used herein, the term "target site" refers to a nucleotide sequence of a portion of DNA of a target gene (for example, the FUT8 gene) to which the ZFP or ZFN described herein is to bind.

In some embodiments, the target site may comprise exon 3 of the FUT8 gene (the relevant sequence is set forth in SEQ ID NO: 1). In some embodiments, the target site may comprise exon 10 of the FUT8 gene and a region adjacent thereto (the relevant sequence is set forth in SEQ ID NO: 2).

As used herein, the zinc fingers in a ZFP are named in the manner of "F" followed by a number sequentially from the N-terminus to the C-terminus. For example, for a given ZFP, sequentially from the N-terminus to the C-terminus, the first zinc finger is named "F1", the second zinc finger is named "F2", and so on.

In some embodiments, one or more zinc fingers of the ZFP have engineered zinc finger recognition regions. In some embodiments, the ZFP has the amino acid sequences of the recognition regions of the zinc fingers (F1 to F5 or F1 to F6) shown in the same row in Table 1:

**Table 1. Zinc finger proteins targeting the FUT8 gene**

| ZFP Name Target Sequence (5'-3') | F1 | F2 | F3 | F4 | F5 | F6 |
|---|---|---|---|---|---|---|
| 32-L1 AATAAGGTCCCCCAGG CA (SEQ ID NO: 7) | QLATLN R (SEQ ID NO: 13) | TRWNLR A (SEQ ID NO: 14) | SRRDLRR (SEQ ID NO: 15) | WRRRLL S (SEQ ID NO: 16) | RSDYLTN (SEQ ID NO: 17) | FHSNLLA (SEQ ID NO: 18) |
| 32-R1 ATATAGGTGGTCATTT GG (SEQ ID NO: 8) | RKSHLT M (SEQ ID NO: 19) | FHSGLLA (SEQ ID NO: 20) | WRRRLL S (SEQ ID NO: 16) | RKYVLL R (SEQ ID NO: 21) | RKDYLV L (SEQ ID NO: 22) | QQAGLIN (SEQ ID NO: 23) |
| L3 AAGAAGGGTCATCAG (SEQ ID NO: 9) | SKWNLR S (SEQ ID NO: 24) | AQSNLLS (SEQ ID NO: 25) | LRHQLR R (SEQ ID NO: 26) | RSDYLTN (SEQ ID NO: 17) | RSDYLTN (SEQ ID NO: 17) | -- |
| R3 AAGGAGGCAAAGACAA AG (SEQ ID NO: 10) | RSDYLT N (SEQ ID NO: 17) | QKITLVR (SEQ ID NO: 27) | RSDYLTN (SEQ ID NO: 17) | QLATLNR (SEQ ID NO: 13) | SRFNLTR (SEQ ID NO: 28) | TKYILTN (SEQ ID NO: 29) |
| S2-L AAGAAGGGTCATCAG (SEQ ID NO: 9) | RADNLT E (SEQ ID NO: 30) | TSGNLTE (SEQ ID NO: 31) | TSGHLVR (SEQ ID NO: 32) | RKDNLK N (SEQ ID NO: 33) | RKDNLK N (SEQ ID NO: 33) | -- |
| S4-R AAGGAGGCAAAGACAA AG (SEQ ID NO: 10) | RSDNLS V (SEQ ID NO: 34) | SPADLTR (SEQ ID NO: 35) | RSDHLSQ (SEQ ID NO: 36) | QSGDLR R (SEQ ID NO: 37) | RSDNLVR (SEQ ID NO: 38) | RKDNLK N (SEQ ID NO: 33) |
| 31-L1 GAACCAAATGACCACC TA (SEQ ID NO: 11) | DRSNLLS (SEQ ID NO: 39) | NQSNLL R (SEQ ID NO: 40) | FHSNLLA (SEQ ID NO: 18) | QLSTLNY (SEQ ID NO: 41) | QSGNLSR (SEQ ID NO: 42) | -- |
| 31-R1 ATGACCACCCTGACCA TT (SEQ ID NO: 12) | FHSGLLA (SEQ ID NO: 20) | HPSTLSK (SEQ ID NO: 43) | ARWTLD C (SEQ ID NO: 44) | HPSTLSK (SEQ ID NO: 43) | HPSTLSK (SEQ ID NO: 43) | RKFTLTN (SEQ ID NO: 45) |

In Table 1, each row describes a single ZFP. The name and DNA target sequence (in the 5'-3' direction) of each ZFP are shown in column 1, and columns 2 to 6 or 7 show the amino acid sequences of the recognition regions of the zinc fingers (F1 to F5 or F1 to F6) in each ZFP.

In some embodiments, the multiple zinc fingers of the ZFP may be linked by suitable linker sequences. In some embodiments, the linker sequence may comprise a linker having a length of 1, 2, 3, 4, 5, or more amino acids.

Further provided herein is a ZFP pair comprising a left ZFP and a right ZFP. The target site of the left ZFP and the target site of the right ZFP in the ZFP pair are located at closely spaced positions on opposite strands (for example, separated by 5 to 10 nucleotide base pairs) of the target gene DNA sequence. For example, the left ZFP in the ZFP pair is capable of binding to a target site on one strand (for example, the template strand or the coding strand) of the target gene DNA sequence, and the right ZFP is capable of binding to a target site on the opposite strand (for example, a coding strand or a template strand) of the target gene DNA sequence located near the target site of the left ZFP (for example, separated by 5 to 20 nucleotide base pairs). In some embodiments, the target site of the left ZFP and the target site of the right ZFP are separated by 5 to 20 nucleotide base pairs, for example, by 5, 6, 7, 8, 9, 10, 11, 12, 13, 14, 15, 16, 17, 18, 19, or 20 nucleotide base pairs.

In some embodiments, the left ZFP may have the following amino acid sequences of the recognition regions (ZFP 32-L1):
F1: QLATLNR (SEQ ID NO: 13);
F2: TRWNLRA (SEQ ID NO: 14);
F3: SRRDLRR (SEQ ID NO: 15);
F4: WRRRLLS (SEQ ID NO: 16);
F5: RSDYLTN (SEQ ID NO: 17), and
F6: FHSNLLA (SEQ ID NO: 18).

In some embodiments, the left ZFP may have the following amino acid sequences of the recognition regions (ZFP L3):
F1: SKWNLRS (SEQ ID NO: 24);
F2: AQSNLLS (SEQ ID NO: 25);
F3: LRHQLRR (SEQ ID NO: 26);
F4: RSDYLTN (SEQ ID NO: 17);
F5: RSDYLTN (SEQ ID NO: 17).

In some embodiments, the left ZFP may have the following amino acid sequences of the recognition regions (ZFP S2-L):
F1: RADNLTE (SEQ ID NO: 30);
F2: TSGNLTE (SEQ ID NO: 31);
F3: TSGHLVR (SEQ ID NO: 32);
F4: RKDNLKN (SEQ ID NO: 33); and
F5: RKDNLKN (SEQ ID NO: 33).

In some embodiments, the left ZFP may have the following amino acid sequences of the recognition regions (ZFP 31-L1):
F1: DRSNLLS (SEQ ID NO: 39);
F2: NQSNLLR (SEQ ID NO: 40);
F3: FHSNLLA (SEQ ID NO: 18);
F4: QLSTLNY (SEQ ID NO: 41); and
F5: QSGNLSR (SEQ ID NO: 42).

In some embodiments, the right ZFP may have the following amino acid sequences of the recognition regions (ZFP 32-R1):
F1: RKSHLTM (SEQ ID NO: 19);
F2: FHSGLLA (SEQ ID NO: 20);
F3: WRRRLLS (SEQ ID NO: 16);
F4: RKYVLLR (SEQ ID NO: 21);
F5: RKDYLVL (SEQ ID NO: 22); and
F6: QQAGLIN (SEQ ID NO: 23).

In some embodiments, the right ZFP may have the following amino acid sequences of the recognition regions (ZFP R3):
RSDYLTN (SEQ ID NO: 17);
QKITLVR (SEQ ID NO: 27);
RSDYLTN (SEQ ID NO: 17);
QLATLNR (SEQ ID NO: 13);
SRFNLTR (SEQ ID NO: 28); and
TKYILTN (SEQ ID NO: 29).

In some embodiments, the right ZFP may have the following amino acid sequences of the recognition regions (ZFP S4-R):
RSDNLSV (SEQ ID NO: 34);
SPADLTR (SEQ ID NO: 35);
RSDHLSQ (SEQ ID NO: 36);
QSGDLRR (SEQ ID NO: 37);
RSDNLVR (SEQ ID NO: 38); and
RKDNLKN (SEQ ID NO: 33).

In some embodiments, the right ZFP may have the following amino acid sequences of the recognition regions (ZFP 31-R1):
FHSGLLA (SEQ ID NO: 20);
HPSTLSK (SEQ ID NO: 43);
ARWTLDC (SEQ ID NO: 44);
HPSTLSK (SEQ ID NO: 43);
HPSTLSK (SEQ ID NO: 43); and
RKFTLTN (SEQ ID NO: 45).

In some embodiments, the ZFP pair may consist of ZFP 32-L1 and ZFP 32-R1. In some embodiments, the ZFP pair may consist of ZFP L3 and ZFP R3. In some embodiments, the ZFP pair may consist of ZFP S2-L and S4-R. In some embodiments, the ZFP pair may consist of ZFP 31-L1 and ZFP 31-R1.

### B. Zinc Finger Nuclease

In another aspect, provided herein is a zinc finger nuclease (ZFN) targeting the FUT8 gene, which is a fusion protein comprising the ZFP described herein and at least one DNA cleavage domain or cleavage half-domain.

As used herein, the term "fusion protein" refers to a protein molecule formed by linking (preferably covalently linking) two or more subunit molecules (for example, the ZFP described herein and a DNA cleavage domain or cleavage half-domain) to each other.

As used herein, the term "cleavage" refers to breakage of the covalent backbone of a DNA molecule. Cleavage may be initiated by various methods, including, but not limited to, enzymatic or chemical hydrolysis of phosphodiester bonds. In some embodiments, cleavage may be single-stranded cleavage or double-stranded cleavage. In some embodiments, double-stranded cleavage may result from two distinct single-stranded cleavage events. Cleavage of DNA may produce blunt ends or staggered ends. In certain embodiments, the ZFN is used for targeted double-stranded DNA cleavage.

As used herein, the term "cleavage half-domain" is a polypeptide sequence that can associate with another polypeptide (the same or different) to form a complex having cleavage activity (preferably double-stranded cleavage activity). In some embodiments, two cleavage half-domains usually cleave in a dimerized form (as a pair).

In some embodiments, the ZFP and at least one DNA cleavage domain or cleavage half-domain are operably linked.

As used herein, the term "operably linked" may mean that one component and other component(s) are connected to each other in a functional manner. For example, with respect to a fusion protein in which a ZFP is fused to a cleavage domain, if the ZFP in the fusion protein can bind its target site and/or its binding site, and the cleavage domain can cleave DNA near the target site, the ZFP and the cleavage domain are operably linked.

The zinc finger nuclease described herein may be used as a tool for targeted cleavage and inactivation of a target gene (such as the FUT8 gene), achieving targeted knockout of the target gene by introducing a double-stranded DNA break (DSB) at a target site of the target gene followed by non-homologous end joining (NHEJ)-mediated DSB repair.

Further provided herein is a ZFN pair comprising a left ZFN and a right ZFN. The left ZFN and the right ZFN are each formed by fusing a ZFP described herein to at least one DNA cleavage domain or cleavage half-domain. For example, ZFN 32-L1 is a fusion protein comprising ZFP 32-L1 and at least one DNA cleavage domain or cleavage half-domain.

The target site of the left ZFN and the target site of the right ZFN in the ZFN pair are located on opposite strands in close proximity (for example, separated by 5 to 10 nucleotide base pairs) on the DNA sequence of the target gene. For example, the left ZFN in the ZFN pair is capable of binding to a target site on one strand (for example, the template strand or the coding strand) of the target gene DNA sequence, and the right ZFN is capable of binding to a target site on the opposite strand (for example, the coding strand or the template strand) of the target gene DNA sequence located near the target site of the left ZFN (for example, separated by 5 to 20 nucleotide base pairs). In some embodiments, the target site of the left ZFN and the target site of the right ZFN are separated by 5 to 20 nucleotide base pairs, for example, by 5, 6, 7, 8, 9, 10, 11, 12, 13, 14, 15, 16, 17, 18, 19, or 20 nucleotide base pairs.

In some embodiments, the ZFN pair may consist of ZFN 32-L1 and ZFN 32-R1. In some embodiments, the ZFN pair may consist of ZFN L3 and ZFN R3. In some embodiments, the ZFN pair may consist of ZFN S2-L and S4-R. In some embodiments, the ZFN pair may consist of ZFN 31-L1 and ZFN 31-R1.

The cleavage domain portion of the fusion proteins disclosed herein may be obtained from any endonuclease or exonuclease. Exemplary endonucleases from which cleavage domains may be derived include, but are not limited to, restriction endonucleases and homing endonucleases. See, for example, New England Biolabs 2002-2003 Catalog, Beverly, Massachusetts; and Belfort et al. (1997) Nucleic Acids Res. 25:3379-3388. Other enzymes capable of cleaving DNA are known (for example, S1 nuclease; mung bean nuclease; pancreatic DNase I; micrococcal nuclease; yeast HO endonuclease; see also Linn et al. (eds.), Nucleases, Cold Spring Harbor Laboratory Press, 1993). One or more of these enzymes (or functional fragments thereof) may be used as a source of cleavage domains and cleavage half-domains.

Similarly, a cleavage half-domain may be derived from any nuclease or fragment thereof, that requires dimerization for its cleavage activity, as mentioned above. Generally, if a fusion protein comprises a cleavage half-domain, two fusion proteins are required to perform cleavage. Optionally, a single protein comprising two cleavage half-domains may be used. The two cleavage half-domains may be derived from the same endonuclease (or a functional fragment thereof), or each cleavage half-domain may be derived from different endonucleases (or a functional fragment thereof). In addition, the target sites of the two fusion proteins are arranged such that binding of the two fusion proteins to their respective target sites places the cleavage half-domains in a spatial orientation relative to each other that allows the cleavage half-domains to form a functional cleavage domain (for example, by dimerization). Thus, in certain embodiments, the target sites may be separated by 5-20 or more nucleotide base pairs. Generally, the cleavage site is located between the target sites.

Restriction endonucleases (restriction enzymes) are present in many species and can bind DNA in a sequence-specific manner (at recognition sites) and cleave DNA at or near the binding sites. Certain restriction enzymes (for example, Type IIS) cleave DNA at sites far away from the recognition site and have separable binding and cleavage domains. Exemplary Type IIS restriction endonucleases are described in International Publication WO 07/014275, which is incorporated herein by reference in its entirety. Thus, in an embodiment, the fusion protein comprises the zinc finger protein and a cleavage domain (or cleavage half-domain) from at least one Type IIS restriction enzyme.

The cleavage domain or cleavage half-domain may be any portion of a protein that retains cleavage activity or retains the ability to multimerize (e.g., dimerize) to form a functional cleavage domain.

In some embodiments, the Type IIS restriction enzyme is FokI. This enzyme is active in dimeric form. Accordingly, in some embodiments, a portion of the FokI enzyme used in the fusion protein functions as a cleavage half-domain. Thus, for targeted double-stranded cleavage and/or targeted replacement of cellular sequences using zinc finger protein-FokI fusions, two fusion proteins, each comprising a FokI cleavage half-domain, may be used to reconstitute a catalytically active cleavage domain. Optionally, a single polypeptide molecule comprising a zinc finger protein and two FokI cleavage half-domains may also be used. In some embodiments, the cleavage half-domain is a FokI cleavage half-domain. In some embodiments, the cleavage half-domain is a wild-type FokI cleavage half-domain. In other embodiments, the cleavage half-domain is an engineered FokI cleavage half-domain.

In some exemplary embodiments, the cleavage half-domain is derived from a Sharkey FokI nuclease derived from wild-type FokI via amino acid mutation, an exemplary amino acid sequence of which is set forth in SEQ ID NO: 3.

The engineered cleavage half-domains described herein may be prepared using any suitable method, for example, by site-directed mutagenesis.

Other restriction enzymes comprising separable binding domains and cleavage domains are also contemplated herein. See, for example, Roberts et al. (2003) Nucleic Acids Res. 31:418-420.

In some embodiments, the cleavage domain may comprise, for example, one or more engineered cleavage half-domains (also referred to as dimerization domain mutants) that minimize or prevent homodimerization, as described in U.S. Patent Publication Nos. 20050064474, 20060188987 and 20080131962 (the disclosures of all of which are incorporated herein by reference in their entireties). Amino acid residues at positions 446, 447, 479, 483, 484, 486, 487, 490, 491, 496, 498, 499, 500, 531, 534, 537 and 538 of FokI are all target sites for affecting dimerization of the FokI cleavage half-domain.

In some embodiments, exemplary pairs of engineered cleavage half-domains of FokI that form obligate heterodimers include those wherein a first cleavage half-domain comprises mutations of the amino acid residues at positions 490 and 538 of FokI, and a second cleavage half-domain comprises mutations of amino acid residues at positions 486 and 499.

In an embodiment, the mutation at position 490 substitutes Lys (K) for Glu (E); the mutation at position 538 substitutes Lys (K) for Ile (I); the mutation at position 486 substitutes Glu (E) for Gln (Q); and the mutation at position 499 substitutes Lys (K) for Iso (I). Specifically, the engineered cleavage half-domains described herein are prepared by mutating the amino acid residues at positions 490 (E→K) and 538 (I→K) in one cleavage half-domain to produce an engineered cleavage half-domain designated "E490K:I538K", and by mutating the amino acid residues at positions 486 (Q→E) and 499 (I→L) in another cleavage half-domain to produce an engineered cleavage half-domain designated "Q486E:I499L". The engineered cleavage half-domains described herein are obligate heterodimer mutants that minimize or eliminate aberrant cleavage when one or more pairs of nucleases comprising these cleavage half-domains are used for cleavage. See, for example, U.S. Patent Publication No. 20080131962, the disclosure of which is incorporated by reference in its entirety for all purposes.

### C. Delivery

The ZFN described herein may be delivered to a cell to inactivate the FUT8 gene in the cell. Delivery of the ZFN into the cell may be achieved by introducing into the cell a polynucleotide encoding the ZFN, wherein the polynucleotide is transcribed in the cell, and the transcript is translated to produce the ZFN fusion protein. Expression of proteins in a cell may further involve trans-splicing, polypeptide cleavage and polypeptide ligation.

In another aspect, provided herein is a polynucleotide encoding the ZFP or ZFN described herein. In some embodiments, the polynucleotide may be mRNA.

The ZFN described herein may be delivered to a target cell by a variety of suitable methods.

In some embodiments, a vector (or vector system) may be used to deliver the ZFN to a target cell.

In another aspect, provided herein is a vector comprising the polynucleotide described above. Useful vectors include, but are not limited to one or more of plasmid vectors, retroviral vectors, lentiviral vectors, adenoviral vectors, adeno-associated viral vectors, poxvirus vectors, herpesvirus vectors, and the like.

In some embodiments, the vector may comprise a polynucleotide encoding one or more ZFNs. In some embodiments, the polynucleotide encoding one or more ZFNs may be located on the same vector or on different vectors. In embodiments employing multiple vectors, each vector may comprise a polynucleotide encoding one or more ZFNs. In embodiments employing multiple vectors, the vectors may be delivered to a target cell simultaneously or sequentially.

In some embodiments, a non-vector delivery method may be used to deliver the ZFN to a target cell. Useful non-vector delivery methods include, but are not limited to, electroporation, lipofection, microinjection, gene gun delivery, liposomes, immunoliposomes, polycation- or lipid-nucleic acid conjugates, naked DNA, agents that enhance DNA uptake. Sonoporation utilizing, for example, the Sonitron 2000 system (Rich-Mar) may also be used to deliver the polynucleotide encoding the ZFN.

Another aspect provided herein is an isolated cell (e.g., a host cell) comprising a ZFP, ZFN, polynucleotide or vector described herein.

Suitable cells include, but are not limited to, eukaryotic and prokaryotic cells and/or cell lines. Non-limiting examples of such cells or cell lines include: COS, CHO (e.g., CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (e.g., HEK293-F, HEK293-H, HEK293-T) and perC6 cells, as well as insect cells such as *Spodoptera frugiperda* (Sf) cells, or fungal cells such as *Saccharomyces cerevisiae, Pichia pastoris* and *Schizosaccharomyces pombe.* In some embodiments, the cell is a mammalian cell. In a particular embodiment, the cell is a Chinese hamster ovary cell (CHO cell). Progeny, variants and derivatives of these cell lines may also be used.

Another aspect provided herein is a cell line comprising a ZFP, ZFN, polynucleotide or vector described herein, wherein the FUT8 gene is inactivated or partially inactivated in the cell line.

Also provided herein is a cell line generated using a ZFP, ZFN, polynucleotide and/or vector described herein, in which the FUT8 gene is inactivated or partially inactivated.

Also provided herein is a FUT8-deficient cell line comprising, at the FUT8 locus, one or more of the nucleotide sequences set forth in SEQ ID NO: 52, SEQ ID NO: 53, SEQ ID NO: 54, SEQ ID NO: 55, SEQ ID NO: 56, SEQ ID NO: 57, SEQ ID NO: 58 and SEQ ID NO: 59.

In some embodiments, the FUT8-deficient cell line comprises, at the FUT8 locus, the nucleotide sequences set forth in SEQ ID NO: 52 and SEQ ID NO: 53. In some embodiments, the FUT8-deficient cell line comprises, at the FUT8 locus, the nucleotide sequences set forth in SEQ ID NO: 54 and SEQ ID NO: 55. In some embodiments, the FUT8-deficient cell line comprises, at the FUT8 locus, the nucleotide sequences set forth in SEQ ID NO: 56 and SEQ ID NO: 57. In some embodiments, the FUT8-deficient cell line comprises, at the FUT8 locus, the nucleotide sequences set forth in SEQ ID NO: 58 and SEQ ID NO: 59.

Also provided herein is a FUT8-deficient cell line comprising an engineered FUT8 locus, wherein the engineered FUT8 locus can be detected using the following primer pairs:
primer pair (i):
   forward primer: AGCCTGAAGTACATAGCCGA (SEQ ID NO: 46); and
   reverse primer: TGCCACTGCTTCTATATACTGATTC (SEQ ID NO: 47); and/or
primer pair (ii):
   forward primer: GACGCACTGACAAAGTGGGA (SEQ ID NO: 48); and
   reverse primer: GGTCTGTTCCATCCCCAGAATG (SEQ ID NO: 49);
   and/or
primer pair (iii):
   forward primer: CTGTTGATTCCAGGTTCCCA (SEQ ID NO: 50); and
   reverse primer: TGTTACTTAAGCCCCAGGC (SEQ ID NO: 51).

In some embodiments, the detection uses a corresponding cell line that is FUT8-normal (non-deficient) as a control cell line. In some embodiments, the assay shows a difference in results between the FUT8-deficient cell line and the control cell line (e.g., a difference in molecular size and band intensity as determined by electrophoresis).

### D. Applications

The ZFN described herein may be used to inactivate the FUT8 gene in a cell. In some embodiments, inactivation includes partial or complete inhibition of expression of the FUT8 gene in the cell. Inactivation of the FUT8 gene may be achieved, for example, by a single cleavage event, by cleavage followed by non-homologous end joining, by cleavage at two sites followed by joining so as to delete the sequence between the two cleavage sites, by targeted integration of a missense or nonsense codon into a coding region via recombination, by targeted recombination of an unrelated sequence (i.e., a "stuffer" sequence) into the gene or the regulatory region thereof so as to disrupt the gene or the regulatory region, or by targeted recombination of a splice acceptor sequence into an intron to cause aberrant splicing of the transcript.

The ZFN-mediated inactivation (knockout or inhibition) described herein has a variety of applications.

Another aspect provided herein is a method for producing a FUT8-deficient cell, the method comprising:
(a) introducing into a cell a polynucleotide encoding one or more ZFNs, wherein each of the ZFNs comprises: (i) a ZFP described herein, which is capable of binding to a target site in the FUT8 gene in the cell; and (ii) a DNA cleavage domain or cleavage half-domain;
(b) allowing the ZFN to be expressed in the cell, such that the ZFN binds to the target site and cleaves the FUT8 gene.

In some embodiments, the one or more ZFNs comprise a ZFN pair described herein.

In some embodiments, the method may further comprise introducing into the cell an additional nuclease that recognizes a target site in the FUT8 gene. Examples of the additional nuclease may include homing endonucleases and meganucleases.

Another aspect provided herein is a method for producing a FUT8-deficient cell line, the method comprising:
(a) inactivating the endogenous FUT8 gene in a cell by the method for producing a FUT8-deficient cell as described herein; and
(b) culturing the cell under conditions suitable for producing a FUT8-deficient cell line.

Exemplary homing endonucleases include I-*Sce* I, I-*Ceu* I, PI-*Psp* I, PI-*Sce*, I-*Sce* IV, I-*Csm* I, I-*Pan* I, I-*Sce* II, I-*Ppo* I, I-*Sce* III, I-*Cre* I, I-*Tev* I, I-*Tev* II and I-*Tev* III. Their recognition sequences are known. See also U.S. Patent No. 5,420,032; U.S. Patent No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalog.

Although the cleavage specificity of most homing endonucleases for their recognition sites is not absolute, these sites are of sufficient length such that a single cleavage event per mammalian-sized genome can be achieved by expressing a homing endonuclease in a cell containing a single copy of the corresponding recognition site. It has also been reported that the specificity of homing endonucleases and meganucleases can be engineered such that they bind non-natural target sites. See, for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66.

Also provided herein is a FUT8-deficient cell line prepared by the method for producing a FUT8-deficient cell line described herein.

Another aspect herein provides a method for culturing a FUT8-deficient cell line, comprising:
(a) providing a FUT8-deficient cell line obtained as described herein; and
(b) culturing or expanding the cells of the cell line.

Yet another aspect herein provides a method for producing a recombinant protein of interest in a host cell, the method comprising:
(a) providing a FUT8-deficient cell described herein,
(b) introducing into the cell an expression vector comprising a nucleic acid encoding the recombinant protein of interest, thereby producing the recombinant protein of interest.

Another aspect herein provides a method for producing a recombinant protein of interest in a host cell, the method comprising:
(a) providing a cell comprising endogenous FUT8 gene;
(b) inactivating the endogenous FUT8 gene in the cell by the method for producing a FUT8-deficient cell as described herein; and
(c) introducing into the cell an expression vector comprising a nucleic acid encoding the recombinant protein of interest, thereby producing the recombinant protein of interest.

In some embodiments, the step of inactivating the endogenous FUT8 gene in the cell as described herein and the step of introducing the expression vector comprising the nucleic acid encoding the recombinant protein of interest into the cell may be performed in either an interchangeable order or simultaneously.

Suitable cells include, but are not limited to, eukaryotic and prokaryotic cells and/or cell lines. Non-limiting examples of such cells or cell lines include: COS, CHO (for example, CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (for example, HEK293-F, HEK293-H, HEK293-T) and perC6 cells, as well as insect cells such as Spodoptera frugiperda (Sf) cells or fungal cells such as *Saccharomyces cerevisiae, Pichia pastoris* and *Schizosaccharomyces pombe.* In some embodiments, the cell is a mammalian cell. In a particular embodiment, the cell is a Chinese hamster ovary cell (CHO cell). Progeny, variants and derivatives of these cell lines may also be used. In some embodiments, the cell is a mammalian cell. In a particular embodiment, the cell is a Chinese hamster ovary cell (CHO cell).

In some embodiments, the target recombinant protein comprises an Fc-containing recombinant protein. In some embodiments, the target recombinant protein is a defucosylated Fc-containing recombinant protein.

Another aspect herein provides the use of the ZFP, ZFN, polynucleotide or vector described herein for producing a FUT8-deficient cell.

Another aspect herein provides the use of the ZFP, ZFN, polynucleotide, vector, cell or cell line described herein for producing a defucosylated Fc-containing recombinant protein.

Another aspect herein provides the use of the ZFP, ZFN, polynucleotide, vector, cell or cell line described herein for producing an antibody (such as a monoclonal antibody) capable of inducing an enhanced ADCC effect.

In some embodiments, the Fc-containing recombinant protein may be an antibody (for example, a monoclonal antibody or a polyclonal antibody) or an Fc fusion protein. The defucosylated Fc-containing recombinant protein provided by the method described herein exhibits an enhanced effector function, particularly in induction of ADCC.

### Examples

The technical solutions of the present invention will be described in more detail below with reference to specific examples. The following examples are merely illustrative and do not constitute any limitation or restriction on the technical solutions of the present invention. Specific technical parameters such as materials, steps, conditions, values or numerical ranges in the following examples are merely illustrative and are neither exhaustive nor limiting.

In addition to the specific methods, equipment and materials used in the examples, based on the knowledge of those skilled in the art regarding the prior art and the disclosure of the present invention, any methods, devices and materials in the prior art that are similar or equivalent to the methods, devices and materials described in the examples of the present invention may also be used to implement the present invention.

The devices and reagents used in the examples are listed in Table 2 and Table 3, respectively.

**Table 2: List of Experimental Equipment**

| Device Name | Device Manufacturer | Model |
|---|---|---|
| Biorad Electroporator | Bio-Rad | Gene Pulser 1652661 |
| Shaker | Kuhner | SMX1503C |
| Electroporation cuvette | Bio-Rad | Gene Pulser Cuvettes 0.4 cm |
| Biosafety cabinet | AIRTECH | BSC-1300 II A2 |
| Benchtop centrifuge | Eppendorf | 5424R |
| Gel imaging system | Bio-Rad | GELDOC XR+ |
| Electrophoresis apparatus | Invitrogen | ZP10002 |
| UV spectrophotometer | THERMO SCIENTIFIC | ND2000 |
| 96-well cell plate | CORNING | 3599 |
| 24-well cell plate | GREINER | 662102 |
| Cell Viability Analyzer | BECKMAN COULTER | Vi-CELL XR |
| Centrifuge | EPPENDORF | 5810R |
| Carbon dioxide incubator | THERMO SCIENTIFIC | HERA cell 240i |
| Microplate Reader | MOLECULAR DEVICE | i3x, M5e |

**Table 3: List of Experimental Reagents**

| Name | Grade | Manufacturer/Supplier | Batch/Catalog No. |
|---|---|---|---|
| CHO-K1 host cells | GMP | WuXi Bio | Lot: CB2020009 |
| DPBS solution | R&D | Corning, Cat#21-031-V | 03322003 |
| T7 High Efficiency Transcription Kit | R&D | Invitrogen, Cat# AM1344 | Lot#01149211 |
| DPBS solution | R&D | HyClone | 21-031-CVC, |
| CD CHO AGT^{™} | R&D | Gibco | 12490-003 |
| ActiPro^{™} | R&D | HyClone | SH31037 |
| Cell Boost 7a | R&D | HyClone | SH31026 |
| Cell Boost 7b | R&D | HyClone | SH31027 |
| Zeocin | R&D | Thermo | R25005, 2417496 |
| Blasticidin | R&D | Gibco | A11139-03, 965513 |
| Lentil lectin | R&D | Medicago | 264427-01 |
| HT1080-TNFα cells | R&D | WuXi Bio | NA |
| Peripheral blood mononuclear cells (PBMC) | R&D | Saili Bio | NA |
| DELFIA Lysis Buffer | R&D | Perkin Elmer | 4005-0010 |
| BATDA Reagent | R&D | Perkin Elmer | C136-100 |
| DELFIA Europium Solution | R&D | Perkin Elmer | C135-100 |

### Example 1 Application of the FUT8^{-/-} Cell Line in Developing the Stable Expression of a Monoclonal Antibody Molecule Adalimumab

### 1 Construction of Expression Vectors

1.1 As exemplarily shown in Figure 2A, the ZFP proteins L3/R3 and S2-L/S4-R were grouped into pairs and were respectively constructed on expression vectors. Transcription was driven by the CMV promoter, and the ZFP protein, as a DNA-binding domain, linked to the downstream FokI cleavage half-domain to form a complete protein, was expressed from the same ORF. Since this expression vector was not subjected to screening after being transfected into CHO cells, no eukaryotic selectable marker was inserted during plasmid construction. Figure 2B shows an exemplary sequence structure, in which 5-6 zinc finger recognition regions were inserted in sequence into a natural zinc finger protein framework sequence (such as ZIF268 or SP1C) and were linked at the C-terminus to a FokI cleavage half-domain. The full-length sequences of S2-L-FokI and S4-R-FokI used in this example were exemplarily shown as SEQ ID NO: 60 and SEQ ID NO: 61. The other ZFP proteins used were all constructed in a similar manner by constructing the corresponding zinc finger recognition regions into the same framework sequence and linking them to FokI.

1.2 The HC sequence (SEQ ID NO: 4) of adalimumab was cloned into expression vector pWX039-HC-Z-105 and linked to the Zeocin resistance gene through EMCV IRES. The LC sequence (SEQ ID NO: 5) of adalimumab was cloned into expression vector pWX040-LC-B-105, placed upstream of the Blasticidin resistance gene. The LC and resistance gene elements belonged to two independent reading frames, and the Blasticidin resistance gene was driven by the SV40 promoter for expression.

### 2 Cell Transfection and Cell Pool Screening

2.1 A suspension of 1E7 CHO-K1 host cells was taken, 30 µg of plasmid was added to 250 µL of DPBS solution and mixed uniformly, and the plasmid solution and cell suspension were thoroughly mixed and then added to an electroporation cuvette. The electroporation cuvette was placed in the slot of a Biorad electroporator, and electroporation was performed according to the corresponding program for the host cells. After transfection, the cell suspension was aspirated from the electroporation cuvette and transferred to a T25 cell culture flask containing 7.5 mL of pre-warmed medium without selection pressure, and cultured statically in a carbon dioxide incubator at 36.5°C, 5% CO₂ and 85% humidity.

2.2 At 48 hours after transfection, the T25 culture flask was transferred to a carbon dioxide incubator at 36.5°C. After another 24 hours of static culture, the cell pool was sampled and counted, and 1E6 cells were taken for extraction of genomic DNA, followed by quantification using a micro UV spectrophotometer. 100 ng to 200 ng of genomic DNA was used, and the target gene sequence was amplified by PCR using a high-fidelity DNA polymerase and suitable primers (primer sequences: Primer 2F: CTGTTGATTCCAGGTTCCCA (SEQ ID NO 50); Primer R: TGTTACTTAAGCCCCAGGC (SEQ ID NO 51)). Then, the reaction was re-denatured and annealed. After annealing was completed, 1 µL of T7E1 enzyme (10 U/µL) was added to the reaction, and the system was incubated at 37°C for 15 minutes. After enzyme digestion, 2% agarose gel electrophoresis was performed to measure the digestion bands and determine the editing efficiency.

### 3 Clone Screening

A cell pool whose viability had recovered to above 90% was subjected to single-cell sorting by the limiting dilution method. After the cells were statically cultured in a 96-well plate for about 2 weeks, the clones were transferred to a 24-well plate. After culturing for about 2-3 days, the clones were expanded into shake tubes for culture, and meanwhile, whether the sequence of the FUT8 gene in the cell line had been knocked out was examined by the NGS method. Passage was performed in the shake tubes every 2-4 days, under culture conditions of 36.5°C, 6% CO₂ and 85% humidity, at a rotational speed of 225 rpm.

### 4 Application of the Target Cell Line in Expressing a Monoclonal Antibody Molecule

4.1 After the screened target cell lines (monoclone 154-F8ZFN-09-08 and monoclone 154-F8ZFN-10-02 were selected in this example) recovered, the target cell lines and a cell line in which the FUT8 gene had not been knocked out (negative control group) were respectively used as host cells. 1E7 cells and 20 µg of adalimumab monoclonal antibody HC and LC plasmids were taken. After the cells and plasmid solution were mixed uniformly, they were transferred to an electroporation cuvette, and electroporated using a Biorad electroporator with suitable parameters selected according to the corresponding cell type. After electroporation, all cells were transferred to a shake tube containing pre-warmed medium and placed in a shaker for culture, under culture conditions of 36.5°C, 6% CO₂ and 85% humidity, at a rotational speed of 225 rpm.

4.2 At 24 hours after transfection, selective medium was added and culture was continued, with passage every 2-4 days.

4.3 After continuous passage for 3-4 weeks, when the viability of the cell pool had recovered to above 95%, the cells were inoculated into production medium at a cell density of 4E5/mL for fed-batch culture. Samples were taken on days 3, 5, 7, 9 and 11 of culture to measure viable cell density and viability, and feeding and glucose supplementation were performed. After 14 days of culture, the supernatant was harvested by centrifugation, and the titer of the monoclonal antibody in the supernatant was measured using the ProA-HPLC method. In addition, after subjecting the harvested supernatant samples to one-step ProA purification, SEC-HPLC detection of high molecular weight (HMW) species content, SDS Caliper and N-glycan-LC glycoform analysis were performed. In this example, the negative control group comprised of host cells in which the FUT8 gene had not been knocked out, which were transfected with the monoclonal antibody vector.

### 5 Experimental Results

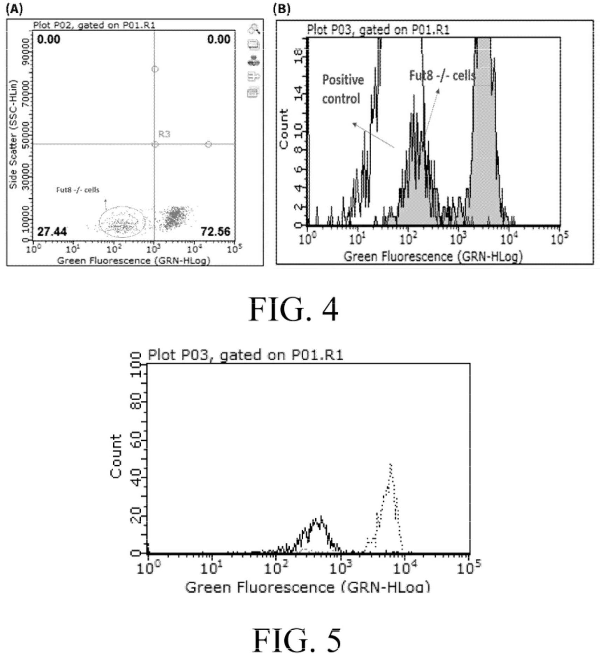

The enzyme digestion experiment results successfully verified that the ZFN plasmid could effectively excise the target gene sequence (Figure 3). The cell pool was subjected to flow cytometric analysis (Figure 4), and a certain proportion of FUT8^{-/-} cell population could be found. Through the clone screening process, clones 154-F8ZFN-09-08 and 154-F8ZFN-10-02 were successfully verified to have one pair of alleles knocked out (Figure 5 and Figure 6). It can be seen from Figures 7A and 7B that the viable cell density and peak VCD of 154-F8ZFN-09-08 and 154-F8ZFN-10-02 during the FB culture process were comparable to those of the control group; Figure 7C shows that there was no significant difference in monoclonal antibody expression titer between the 154-F8ZFN-09-08 group and the control group. In addition, the SEC-HPLC and Caliper R/NR results show that there was no significant difference in the high-polymer level of the one-step purified samples from the cell pools of the 154-F8ZFN-09-08 and 154-F8ZFN-10-02 groups and the control group (Figures 7D and 7E). Finally, it can be seen from the glycoform results (Figure 7F) that the fucose levels of the 154-F8ZFN-09-08 and 154-F8ZFN-10-02 groups were far lower than that of the negative control group in which the FUT8 gene had not been knocked out, decreasing to a level close to zero.

### Example 2 Application of the FUT8^{-/-} Cell Line in Developing the Stable Expression of the Fusion Protein Molecule Dulaglutide

### 1 Construction of Expression Vectors

Dulaglutide is a glucagon-like peptide 1 (GLP-1) Fc fusion protein.

The sequence of Dulaglutide (SEQ ID NO: 6) was cloned into expression vectors pWX039-Pr-Z-126A3 and pWX040-Pr-B-126A3. On vector pWX039-Pr-Z-126A3, the sequence of Dulaglutide was linked to the Zeocin resistance gene through EMCV IRES. On pWX040-Pr-B-126A3, the sequence of Dulaglutide was placed upstream of the Blasticidin resistance gene, the gene of interest and the resistance gene element belonged to two independent reading frames, with the Blasticidin resistance gene being driven by the SV40 promoter for expression.

### 2 Cell Transfection and Fed-batch Culture

2.1 Cells of 154-F8ZFN-09-08 screened in Example 1 were used as the host cells, and 1E7 cells and 20 µg of expression vector were mixed uniformly and then transferred to an electroporation cuvette. Electroporation was performed using a Biorad electroporator by selecting suitable electroporation parameters according to the corresponding cell type. After electroporation, all cells were transferred to a shake tube containing pre-warmed medium and placed in a shaker for culture, under culture conditions of 36.5°C, 6% CO₂ and 85% humidity, at a rotational speed of 225 rpm.

2.2 At 24 hours after transfection, an equal volume of selective medium was added and culture was continued, with passage every 2-4 days.

2.3 After continuous passage for 3-4 weeks, when the viability of the cell pool had recovered to above 95%, the cells were inoculated into production medium at a cell density of 4E5/mL for fed-batch culture. Samples were taken on days 3, 5, 7, 9 and 11 of culture to measure viable cell density and viability, and feeding and glucose supplementation were performed. After 14 days of culture, the supernatant was harvested by centrifugation, and the titer of the monoclonal antibody in the supernatant was measured using the ProA-HPLC method. In addition, after one-step proA purification was performed on the harvested supernatant sample, SEC HPLC detection of high molecular weight (HMW) species content, SDS Caliper NR purity analysis and N-glycan glycoform analysis were performed. In this example, the positive control group comprised of host cells in which the FUT8 gene had been knocked out and which were transfected with the fusion protein vector.

### 3 Experimental Results

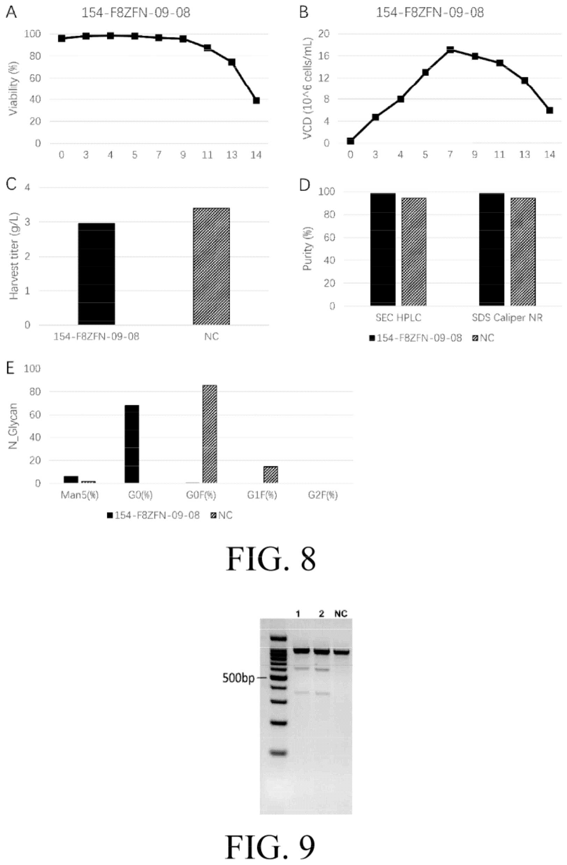

Figure 8A shows that the viable cell density and viability data of the 154-F8ZFN-09-08 group during the FB culture process were within acceptable ranges; Figures 8C, D and E show that the protein expression titer, SEC-HPLC main peak and SDS Caliper NR main peak of the 154-F8ZFN-09-08 group did not differ greatly from those of the control group. Finally, the glycoform results (Figure 8E) show that the fucose level of the 154-F8ZFN-09-08 group was far lower than that of the negative control group in which the FUT8 gene had not been knocked out, decreasing to a level close to zero.

### Example 3 ZFN Editing at Exon 3

### 1. Construction of Expression Vectors

1.1 As exemplarily shown in Figure 2A, similarly to that described in Example 1, the ZFP proteins 31-L1/31-R1 and 32-L1/32-R1 were grouped into pairs and were respectively constructed on expression vectors.

### 2. Cell Transfection and Cell Population (Pool) Screening

2.2 1×10^7 CHO-K1 cells were mixed uniformly with the 32-L1/32-R1 and the 31-L1/31-R1 plasmids, respectively, and transfected in a Bio-rad electroporator using program WE. After transfection, the cells were allowed to recover in 5 mL of CD CHO medium and cultured in a static incubator at 30°C.

2.3 On day 2 after transfection, cells were collected, genomic DNA was prepared, and the targeted region of the FUT8 gene was amplified using primers Fut8-EX3-F1 (SEQ ID NO: 46) and Fut8-EX3-R1 (SEQ ID NO: 47); finally, the gene editing efficiency was detected using a T7E1 kit. As shown in lane 2 of Figure 9, according to the calculation of band brightness by ImageJ, 32-L1/32-R1 modified the FUT8 exon 3 region, resulting in 10.9% DNA mutation; as shown in lane 1, 31-L1/31-R1 modified the FUT8 exon 3 region, resulting in 9.4% DNA mutation.

### 3 Monoclone Screening

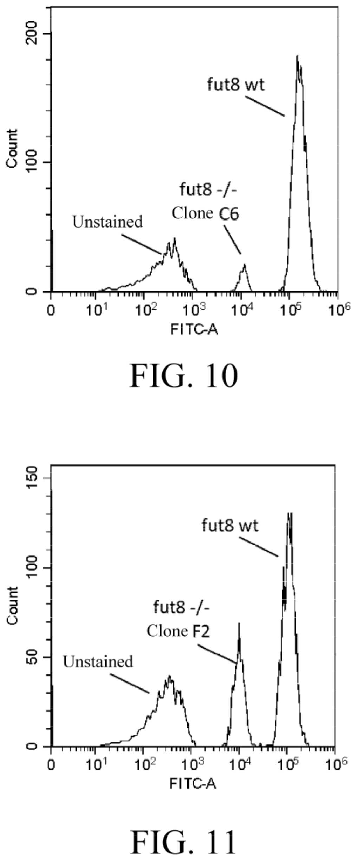

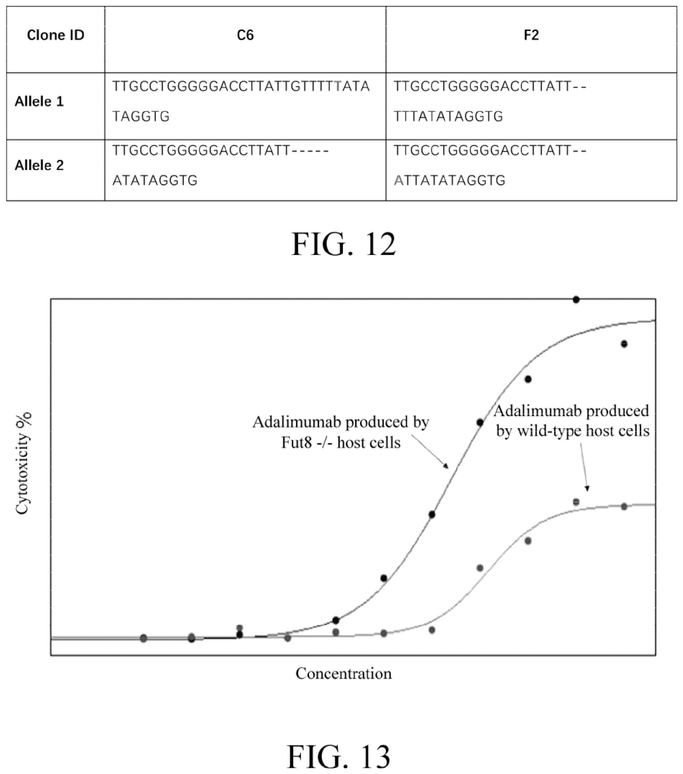

On day 34 after transfection, SCP monoclonal sorting was performed. All grown monoclones were subjected to LCA-FITC staining, and finally two monoclones C6 and F2 with the FUT8^{-/-} phenotype were obtained (Figures 10 and 11). As confirmed by sequence verification, the sequencing results of these two clones are shown in Figure 12, demonstrating that both alleles were knocked out.

### Example 4 Significantly improved ADCC activity of an antibody expressed by a FUT8^{-/-} clone generated by ZFN knockout

### 1. Protein expression

Using a FUT8^{-/-} double-knockout clone (154-F8ZFN-09-08) obtained according to the method described herein as the host cell, and unmutated wild-type CHO as a control, and adalimumab was expressed and purified according to the method described above.

### 2. Target cell labeling

HT1080 cells expressing TNFα were first labeled with DELFIA BATDA (Cat# C136-100, Perkin Elmer, China) for 30 to 40 minutes in a humidified incubator at 37°C and 5% CO₂. After incubation, the HT1080 cells were centrifuged and washed with assay medium, and then resuspended in assay medium (RPMI 1640 + 10% HI-FBS).

### 3. Co-incubation of target cells and effector cells

Peripheral blood mononuclear cells (PBMCs) were resuspended in assay medium. 1×10^6 PBMCs and 1×10^4 HT1080 cells expressing TNFα were thoroughly mixed, anti-TNFα therapeutic antibody samples were added at final concentrations ranging from 0 to 50 ng/mL, and the cells were co-cultured at 200 µL/well in a 96-well plate for 1 to 2 hours in a 37°C, 5% CO₂ incubator.

### 4. Supernatant reaction

After the 96-well plate was centrifuged at 500 × g for 3 to 5 minutes, 20 µL of supernatant was taken from each well and transferred to the corresponding well in a 96-well white microplate (3912, Corning, USA). Subsequently, 200 µL of DELFIA Europium Solution (Cat# C135-100, Perkin Elmer, China) was added to each well, and the plate was incubated for 20 to 30 minutes.

### 5. Data processing

Data were acquired on a microplate reader M5e (Molecular Devices, California, USA). Relative fluorescence units (RFU) were plotted against antibody concentration to generate a 4-parameter logistic response curve. When all system and sample suitability criteria were met, the relative potency of the sample was calculated by the EC50 ratio. As shown in FIG. 13, the ADCC activity of adalimumab produced by the FUT8^{-/-} double-knockout host cells was significantly higher than that of the same protein produced by the control wild-type CHO host.

### Sequence Listing

SEQ ID NO: 1 CHO FUT8 gene exon 3 sequence 
CATGTAGAGCCCAGGAAGCACAGGACAAGAAAGCTGCCTCCTTGTATCACCAG
GAAGATCTTTTTGTAAGAGTCATCACAGTATACCAGAGAGACTAATTTTGTCT
GAAGCATCATGTGTTGAAACAACAGAAACTTATTTCCTGTGTGGCTAACTAG
AACCAGAGTACAATGTTTCCAATTCTTTGAGCTCCGAGAAGACAGAAGGGAGT
TGAAACTCTGAAAATGCGGGCATGGACTGGTTCCTGGCGTTGGATTATGCTCA
TTCTTTTTGCCTGGGGGACCTTATTGTTTTATATAGGTGGTCATTTGGTTCGA
GATAATGACCACCCTGACCATTCTAGCAGAGAACTCTCCAAGATTCTTGCAAA
GCTGGAGCGCTTAAAACAACAAAATGAAGACTTGAGGAGAATGGCTGAGTCTC
TCCG
SEQ ID NO: 2 Nucleotide sequence of CHO FUT8 gene exon 10 and flanking regions (including a portion of intron 10) 
AGTCCATGTCAGACGCACTGACAAAGTGGGAACAGAAGCAGCCTTCCATCCCA
TTGAGGAATACATGGTACACGTTGAAGAACATTTTCAGCTTCTCGAACGCAGA
ATGAAAGTGGATAAAAAAAGAGTGTATCTGGCCACTGATGACCCTTCTTTGTT
AAAGGAGGCAAAGACAAAGTAAGTTAGACCAACAAGTGGTTCTGTATGGGATT
ATCTCTTAGTTGAAGAAAATCCTTAATTCTGGGAACTTGTGGTTCTTGTTGCT
AACTAATAGGTTCCAAAATCAAAGACTACATGTGCAAATATTAATCTAATCAA
GTCATACCTTACTAGCTGTATCTGATGCAAATTAAGAAGTCTAAAATGAAT
SEQ ID NO: 3 Amino acid sequence of restriction endonuclease Sharkey FokI 
QLVKSELEEKKSELRHKLKYVPHEYIELIEIARNPTQDRILEMKVMEFFMKVY
GYRGEHLGGSRKPDGAIYTVGSPIDYGVIVDTKAYSGGYNLPIGQADEMQRYV
EENQTRNKHINPNEWWKVYPSSVTEFKFLFVSGHFKGNYKAQLTRLNHITNCN
GAVLSVEELLIGGEMIKAGTLTLEEVRRKFNNGEINF
SEQ ID NO: 4 Amino acid sequence of adalimumab HC 
MEFGLSWLFLVAILKGVQCEVQLVESGGGLVQPGRSLRLSCAASGFTFDDYAM
HWVRQAPGKGLEWVSAITWNSGHIDYADSVEGRFTISRDNAKNSLYLQMNSLR
AEDTAVYYCAKVSYLSTASSLDYWGQGTLVTVSSASTKGPSVFPLAPSSKSTS
GGTAALGCLVKDYFPEPVTVSWNSGALTSGVHTFPAVLQSSGLYSLSSVVTVP
SSSLGTQTYICNVNHKPSNTKVDKKVEPKSCDKTHTCPPCPAPELLGGPSVFL
FPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQV
 
YTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
SEQ ID NO: 5 Amino acid sequence of adalimumab LC 
MDMRVPAQLLGLLLLWFPGSRCDIQMTQSPSSLSASVGDRVTITCRASQGIRN
YLAWYQQKPGKAPKLLIYAASTLQSGVPSRFSGSGSGTDFTLTISSLQPEDVA
TYYCQRYNRAPYTFGQGTKVEIKRTVAAPSVFIFPPSDEQLKSGTASVVCLLN
NFYPREAKVQWKVDNALQSGNSQESVTEQDSKDSTYSLSSTLTLSKADYEKHK
VYACEVTHQGLSSPVTKSFNRGEC
SEQ ID NO: 6 Amino acid sequence of Dulaglutide 
MPLLLLLPLLWAGALAHGEGTFTSDVSSYLEEQAAKEFIAWLVKGGGGGGGSG
GGGSGGGGSAESKYGPPCPPCPAPEAAGGPSVFLFPPKPKDTLMISRTPEVTC
VVVDVSQEDPEVQFNWYVDGVEVHNAKTKPREEQFNSTYRVVSVLTVLHQDWL
NGKEYKCKVSNKGLPSSIEKTISKAKGQPREPQVYTLPPSQEEMTKNQVSLTC
LVKGFYPSDIAVEWESNGQPENNYKTTPPVLDSDGSFFLYSRLTVDKSRWQEG
NVFSCSVMHEALHNHYTQKSLSLSLG
SEQ ID NO: 7 ZFN 32-L1 target sequence
   ACGGACCCCCTGGAATAA
SEQ ID NO: 8 ZFN 32-R1 target sequence
   GGTTTACTGGTGGATATA
SEQ ID NO: 9 ZFN L3 target sequence, ZFN S2-L target sequence
   GACTACTGGGAAGAA
SEQ ID NO: 10 ZFN R3 target sequence, ZFN S4-R target sequence
   GAAACAGAAACGGAGGAA
SEQ ID NO: 11 ZFN 31-L1 target sequence
   ATCCACCAGTAAACCAAG
SEQ ID NO: 12 ZFN 31-R1 target sequence
   TTACCAGTCCCACCAGTA
SEQ ID NO: 13 Zinc finger recognition region sequence
   QLATLNR
SEQ ID NO: 14 Zinc finger recognition region sequence
   TRWNLRA
SEQ ID NO: 15 Zinc finger recognition region sequence
   SRRDLRR
SEQ ID NO: 16 Zinc finger recognition region sequence
   WRRRLLS
SEQ ID NO: 17 Zinc finger recognition region sequence
   RSDYLTN
SEQ ID NO: 18 Zinc finger recognition region sequence
   FHSNLLA
SEQ ID NO: 19 Zinc finger recognition region sequence
   RKSHLTM
SEQ ID NO: 20 Zinc finger recognition region sequence
   FHSGLLA
SEQ ID NO: 21 Zinc finger recognition region sequence
   RKYVLLR
SEQ ID NO: 22 Zinc finger recognition region sequence
   RKDYLVL
SEQ ID NO: 23 Zinc finger recognition region sequence
   QQAGLIN
SEQ ID NO: 24 Zinc finger recognition region sequence
   SKWNLRS
SEQ ID NO: 25 Zinc finger recognition region sequence
   AQSNLLS
SEQ ID NO: 26 Zinc finger recognition region sequence
   LRHQLRR
SEQ ID NO: 27 Zinc finger recognition region sequence
   QKITLVR
SEQ ID NO: 28 Zinc finger recognition region sequence
   SRFNLTR
SEQ ID NO: 29 Zinc finger recognition region sequence
   TKYILTN
SEQ ID NO: 30 Zinc finger recognition region sequence
   RADNLTE
SEQ ID NO: 31 Zinc finger recognition region sequence
   TSGNLTE
SEQ ID NO: 32 Zinc finger recognition region sequence
   TSGHLVR
SEQ ID NO: 33 Zinc finger recognition region sequence
   RKDNLKN
SEQ ID NO: 34 Zinc finger recognition region sequence
   RSDNLSV
SEQ ID NO: 35 Zinc finger recognition region sequence
   SPADLTR
SEQ ID NO: 36 Zinc finger recognition region sequence
   RSDHLSQ
SEQ ID NO: 37 Zinc finger recognition region sequence
   QSGDLRR
SEQ ID NO: 38 Zinc finger recognition region sequence
   RSDNLVR
SEQ ID NO: 39 Zinc finger recognition region sequence
   DRSNLLS
SEQ ID NO: 40 Zinc finger recognition region sequence
   NQSNLLR
SEQ ID NO: 41 Zinc finger recognition region sequence
   QLSTLNY
SEQ ID NO: 42 Zinc finger recognition region sequence
   QSGNLSR
SEQ ID NO: 43 Zinc finger recognition region sequence
   HPSTLSK
SEQ ID NO: 44 Zinc finger recognition region sequence
   ARWTLDC
SEQ ID NO: 45 Zinc finger recognition region sequence
   RKFTLTN
SEQ ID NO: 46 Fut8-EX3-F1 primer
   AGCCTGAAGTACATAGCCGA
SEQ ID NO: 47 Fut8-EX3-R1 primer
   TGCCACTGCTTCTATATACTGATTC
SEQ ID NO: 48 P5-F primer
   GACGCACTGACAAAGTGGGA
SEQ ID NO: 49 P5-R primer
   GGTCTGTTCCATCCCCAGAATG
SEQ ID NO: 50 2F primer
   CTGTTGATTCCAGGTTCCCA
SEQ ID NO: 51 R primer
   TGTTACTTAAGCCCCAGGC
SEQ ID NO: 52 154-F8ZFN-09-08 allele 1
   CCCTTCTTAAGGAGGCAAAGA
SEQ ID NO: 53 154-F8ZFN-09-08 allele 2
   CCCTTCTAAAGGAGGCAAAGA
SEQ ID NO: 54 154-F8ZFN-10-02 allele 1
   CCCTTCTGTTAAAGGAGGCAAAGA
SEQ ID NO: 55 154-F8ZFN-10-02 allele 2
   CCCTTCTTTAAAGGAGGCAAAGA
SEQ ID NO: 56 C6 allele 1
   TTGCCTGGGGGACCTTATTGTTTTTATATAGGTG
SEQ ID NO: 57 C6 allele 2
   TTGCCTGGGGGACCTTATTATATAGGTG
SEQ ID NO: 58 F2 allele 1
   TTGCCTGGGGGACCTTATTTTTATATAGGTG
SEQ ID NO: 59 F2 allele 2
   TTGCCTGGGGGACCTTATTATTATATAGGTG
SEQ ID NO: 60 Full-length amino acid sequence of ZFP S2-L-FokI 
PKKKRKVLEPGEKPYKCPECGKSFSRADNLTEHQRTHTGEKPYKCPECGKSFS
TSGNLTEHQRTHTGEKPYKCPECGKSFSTSGHLVRHQRTHTGEKPYKCPECGK
SFSRKDNLKNHQRTHTGEKPYKCPECGKSFSRKDNLKNHQRTHTGKKTSGSQL
 
VKSELEEKKSELRHKLKYVPHEYIELIEIARNPTQDRILEMKVMEFFMKVYGY
RGEHLGGSRKPDGAIYTVGSPIDYGVIVDTKAYSGGYNLPIGQADEMQRYVEE
NQTRNKHINPNEWWKVYPSSVTEFKFLFVSGHFKGNYKAQLTRLNHITNCNGA
VLSVEELLIGGEMIKAGTLTLEEVRRKFNNGEINF
SEQ ID NO: 61 Full-length amino acid sequence of ZFP S4-R-FokI

PKKKRKVLEPGEKPYKCPECGKSFSRSDNLSVHQRTHTGEKPYKCPECGKSFS
SPADLTRHQRTHTGEKPYKCPECGKSFSRSDHLSQHQRTHTGEKPYKCPECGK
SFSQSGDLRRHQRTHTGEKPYKCPECGKSFSRSDNLVRHQRTHTGEKPYKCPE
CGKSFSRKDNLKNHQRTHTGKKTSGSQLVKSELEEKKSELRHKLKYVPHEYIE
LIEIARNPTQDRILEMKVMEFFMKVYGYRGEHLGGSRKPDGAIYTVGSPIDYG
VIVDTKAYSGGYNLPIGQADEMQRYVEENQTRNKHINPNEWWKVYPSSVTEFK
FLFVSGHFKGNYKAQLTRLNHITNCNGAVLSVEELLIGGEMIKAGTLTLEEVR
RKFNNGEINF

## Claims

1. A zinc finger protein capable of targeting and binding to the FUT8 gene, comprising, sequentially from the N-terminus to the C-terminus, amino acid sequences of zinc finger recognition regions of zinc fingers F1 to F5 or F1 to F6 shown in the same row of the following table:
| F1 | F2 | F3 | F4 | F5 | |
|---|---|---|---|---|---|
| SKWNLRS (SEQ ID NO: 24) | AQSNLLS (SEQ ID NO: 25) | LRHQLRR (SEQ ID NO: 26) | RSDYLTN (SEQ ID NO: 17) | RSDYLTN (SEQ ID NO: 17) | |
| F1 | F2 | F3 | F4 | F5 | |
|---|---|---|---|---|---|
| RADNLTE (SEQ ID NO: 30) | TSGNLTE (SEQ ID NO: 31) | TSGHLVR (SEQ ID NO: 32) | RKDNLKN (SEQ ID NO: 33) | RKDNLKN (SEQ ID NO: 33) | |
| F1 | F2 | F3 | F4 | F5 | |
|---|---|---|---|---|---|
| DRSNLLS (SEQ ID NO: 39) | NQSNLLR (SEQ ID NO: 40) | FHSNLLA (SEQ ID NO: 18) | QLSTLNY (SEQ ID NO: 41) | QSGNLSR (SEQ ID NO: 42) | |
| F1 | F2 | F3 | F4 | F5 | F6 |
|---|---|---|---|---|---|
| QLATLNR (SEQ ID NO: 13) | TRWNLRA (SEQ ID NO: 14) | SRRDLRR (SEQ ID NO: 15) | WRRRLLS (SEQ ID NO: 16) | RSDYLTN (SEQ ID NO: 17) | FHSNLLA (SEQ ID NO: 18) |
| F1 | F2 | F3 | F4 | F5 | F6 |
|---|---|---|---|---|---|
| RKSHLTM (SEQ ID NO: 19) | FHSGLLA (SEQ ID NO: 20) | WRRRLLS (SEQ ID NO: 16) | RKYVLLR (SEQ ID NO: 21) | RKDYLVL (SEQ ID NO: 22) | QQAGLIN (SEQ ID NO: 23) |
| F1 | F2 | F3 | F4 | F5 | F6 |
|---|---|---|---|---|---|
| RSDYLTN (SEQ ID NO: 17) | QKITLVR (SEQ ID NO: 27) | RSDYLTN (SEQ ID NO: 17) | QLATLNR (SEQ ID NO: 13) | SRFNLTR (SEQ ID NO: 28) | TKYILTN (SEQ ID NO: 29) |
| F1 | F2 | F3 | F4 | F5 | F6 |
|---|---|---|---|---|---|
| RSDNLSV (SEQ ID NO: 34) | SPADLTR (SEQ ID NO: 35) | RSDHLSQ (SEQ ID NO: 36) | QSGDLRR (SEQ ID NO: 37) | RSDNLVR (SEQ ID NO: 38) | RKDNLKN (SEQ ID NO: 33) |
| F1 | F2 | F3 | F4 | F5 | F6 |
|---|---|---|---|---|---|
| FHSGLLA (SEQ ID NO: 20) | HPSTLSK (SEQ ID NO: 43) | ARWTLDC (SEQ ID NO: 44) | HPSTLSK (SEQ ID NO: 43) | HPSTLSK (SEQ ID NO: 43) | RKFTLTN (SEQ ID NO: 45) |

2. A zinc finger nuclease, comprising the zinc finger protein according to claim 1 and at least one DNA cleavage domain or cleavage half-domain.

3. A polynucleotide encoding the zinc finger protein according to claim 1.

4. A polynucleotide encoding the zinc finger nuclease according to claim 2.

5. A vector comprising the polynucleotide according to claim 3 or 4.

6. An isolated cell, comprising the zinc finger protein according to claim 1, the zinc finger nuclease according to claim 2, the polynucleotide according to claim 3 or 4, or the vector according to claim 5.

7. A method for producing a FUT8-deficient cell, the method comprising:
(a) introducing into a cell a polynucleotide encoding one or more zinc finger nucleases according to claim 2;
(b) allowing the zinc finger nuclease to be expressed in the cell, whereby the zinc finger nuclease binds to the target site and cleaves the FUT8 gene.

8. The method according to claim 7, wherein, in step (a), a zinc finger nuclease pair comprising a left zinc finger nuclease and a right zinc finger nuclease is introduced into the cell, wherein the zinc finger nuclease pair comprises:
zinc finger nuclease pair (a):
a left zinc finger nuclease having the following amino acid sequences of zinc finger recognition regions:
F1: QLATLNR (SEQ ID NO: 13);
F2: TRWNLRA (SEQ ID NO: 14);
F3: SRRDLRR (SEQ ID NO: 15);
F4: WRRRLLS (SEQ ID NO: 16);
F5: RSDYLTN (SEQ ID NO: 17); and
F6: FHSNLLA (SEQ ID NO: 18);
and
a right zinc finger nuclease having the following amino acid sequences of zinc finger recognition regions:
F1: RKSHLTM (SEQ ID NO: 19);
F2: FHSGLLA (SEQ ID NO: 20);
F3: WRRRLLS (SEQ ID NO: 16);
F4: RKYVLLR (SEQ ID NO: 21);
F5: RKDYLVL (SEQ ID NO: 22); and
F6: QQAGLIN (SEQ ID NO: 23);
and/or
zinc finger nuclease pair (b):
a left zinc finger nuclease having the following amino acid sequences of zinc finger recognition regions:
F1: SKWNLRS (SEQ ID NO: 24);
F2: AQSNLLS (SEQ ID NO: 25);
F3: LRHQLRR (SEQ ID NO: 26);
F4: RSDYLTN (SEQ ID NO: 17); and
F5: RSDYLTN (SEQ ID NO: 17);
and
a right zinc finger nuclease having the following amino acid sequences of zinc finger recognition regions:
RSDYLTN (SEQ ID NO: 17);
QKITLVR (SEQ ID NO: 27);
RSDYLTN (SEQ ID NO: 17);
QLATLNR (SEQ ID NO: 13);
SRFNLTR (SEQ ID NO: 28); and
TKYILTN (SEQ ID NO: 29);
and/or
zinc finger nuclease pair (c):
a left zinc finger nuclease having the following amino acid sequences of zinc finger recognition regions:
F1: RADNLTE (SEQ ID NO: 30);
F2: TSGNLTE (SEQ ID NO: 31);
F3: TSGHLVR (SEQ ID NO: 32);
F4: RKDNLKN (SEQ ID NO: 33); and
F5: RKDNLKN (SEQ ID NO: 33);
and
a right zinc finger nuclease having the following amino acid sequences of zinc finger recognition regions:
RSDNLSV (SEQ ID NO: 34);
SPADLTR (SEQ ID NO: 35);
RSDHLSQ (SEQ ID NO: 36);
QSGDLRR (SEQ ID NO: 37);
RSDNLVR (SEQ ID NO: 38); and
RKDNLKN (SEQ ID NO: 33);
and/or
zinc finger nuclease pair (d):
a left zinc finger nuclease having the following amino acid sequences of zinc finger recognition regions:
F1: DRSNLLS (SEQ ID NO: 39);
F2: NQSNLLR (SEQ ID NO: 40);
F3: FHSNLLA (SEQ ID NO: 18);
F4: QLSTLNY (SEQ ID NO: 41); and
F5: QSGNLSR (SEQ ID NO: 42);
and
a right zinc finger nuclease having the following amino acid sequences of zinc finger recognition regions:
FHSGLLA (SEQ ID NO: 20);
HPSTLSK (SEQ ID NO: 43);
ARWTLDC (SEQ ID NO: 44);
HPSTLSK (SEQ ID NO: 43);
HPSTLSK (SEQ ID NO: 43); and
RKFTLTN (SEQ ID NO: 45).

9. A method for producing a FUT8-deficient cell line, the method comprising:
(a) inactivating the FUT8 gene in a cell by the method according to claim 7; and
(b) culturing the cell under conditions suitable for producing a FUT8-deficient cell line;
specifically, the cell is a mammalian cell; more specifically, the cell is a CHO cell.

10. A method for producing a recombinant protein of interest in a host cell, the method comprising:
(a) providing the cell according to claim 6, wherein the cell is a FUT8-deficient cell,
(b) introducing into the cell an expression vector comprising a nucleic acid encoding the recombinant protein of interest, thereby producing the recombinant protein of interest;
specifically, the cell is a mammalian cell; more specifically, the cell is a CHO cell.

11. A method for producing a recombinant protein of interest in a host cell, the method comprising:
(a) providing a cell comprising endogenous FUT8 gene;
(b) inactivating the endogenous FUT8 gene in the cell by the method according to claim 7; and
(c) introducing into the cell an expression vector comprising a nucleic acid encoding the recombinant protein of interest, thereby producing the recombinant protein of interest;
more specifically, step (b) is performed before, simultaneously with, or after step (c).

12. Use of the zinc finger protein according to claim 1, the zinc finger nuclease according to claim 2, the polynucleotide according to claim 3 or 4, or the vector according to claim 5 for producing a FUT8-deficient cell.

13. Use of the zinc finger protein according to claim 1, the zinc finger nuclease according to claim 2, the polynucleotide according to claim 3 or 4, the vector according to claim 5, or the cell according to claim 6 for producing a defucosylated Fc-containing recombinant protein.

14. Use of the zinc finger protein according to claim 1, the zinc finger nuclease according to claim 2, the polynucleotide according to claim 3 or 4, the vector according to claim 5, or the cell according to claim 6 for producing an antibody (such as a monoclonal antibody) capable of inducing an enhanced ADCC effect.

15. A FUT8-deficient cell line, prepared by the method according to claim 9.

16. A FUT8-deficient cell line, comprising, at the FUT8 locus, a nucleotide sequence shown in one or more of SEQ ID NO: 52, SEQ ID NO: 53, SEQ ID NO: 54, SEQ ID NO: 55, SEQ ID NO: 56, SEQ ID NO: 57, SEQ ID NO: 58, and SEQ ID NO: 59.

17. A FUT8-deficient cell line, comprising an engineered FUT8 locus, wherein the engineered FUT8 locus can be detected by the following primer pairs:
primer pair (i):
forward primer: AGCCTGAAGTACATAGCCGA (SEQ ID NO: 46); and
reverse primer: TGCCACTGCTTCTATATACTGATTC (SEQ ID NO: 47);
and/or
primer pair (ii):
forward primer: GACGCACTGACAAAGTGGGA (SEQ ID NO: 48); and
reverse primer: GGTCTGTTCCATCCCCAGAATG (SEQ ID NO: 49);
and/or
primer pair (iii):
forward primer: CTGTTGATTCCAGGTTCCCA (SEQ ID NO: 50); and
reverse primer: TGTTACTTAAGCCCCAGGC (SEQ ID NO: 51).

18. A method for culturing a FUT8-deficient cell line, comprising:
(a) providing the FUT8-deficient cell line according to any one of claims 15-17; and
(b) culturing or expanding the cells of the cell line.
